(19) Europäisches Patentamt
European Patent Office
Office européen des brevets

(11) **EP 3 366 643 A1**

(12) **EUROPÄISCHE PATENTANMELDUNG**

(43) Veröffentlichungstag:
**29.08.2018 Patentblatt 2018/35**

(51) Int Cl.:
**C01B 39/14** [(2006.01)]     **C07C 2/12** [(2006.01)]
**B01J 29/76** [(2006.01)]

(21) Anmeldenummer: **18158148.9**

(22) Anmeldetag: **22.02.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(30) Priorität: **27.02.2017   EP 17158066**

(71) Anmelder:
• **Evonik Degussa GmbH**
  **45128 Essen (DE)**
• **Technische Universität München**
  **80333 München (DE)**

(72) Erfinder:
• **Ehrmaier, Andreas**
  **85368 Moosburg (DE)**
• **Bermejo De Val, Ricardo**
  **80807 München (DE)**
• **Sanchez-Sanchez, Maria Cruz**
  **80469 München (DE)**
• **Liu, Yue**
  **82008 Unterhaching (DE)**
• **Lercher, Johannes A.**
  **85521 Ottobrunn (DE)**
• **Peitz, Stephan**
  **45739 Oer-Erkenschwick (DE)**
• **Stochniol, Guido**
  **45721 Haltern am See (DE)**

(54) **SELEKTIVE OLIGOMERISIERUNG VON OLEFINEN**

(57)     Die Erfindung betrifft ein Katalysatorsystem zur Oligomerisierung von Olefinen an einem Nickel enthaltenden Katalysator, die Verwendung dieses Katalysators sowie ein Verfahren zur Dimerisierung von Olefinen.

**EP 3 366 643 A1**

**Beschreibung**

Die Erfindung betrifft ein Katalysatorsystem zur Oligomerisierung von Olefinen an einem Nickel enthaltenden Katalysator, die Verwendung dieses Katalysators sowie ein Verfahren zur Oligomerisierung von Olefinen.

Generell versteht man unter der Oligomerisierung die Reaktion von Kohlenwasserstoffen mit sich selbst, wobei entsprechend längerkettige Kohlenwasserstoffe, die so genannten Oligomere, entstehen. Olefine mit zwei bis acht Kohlenstoffatomen lassen sich recht gut oligomerisieren. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere, insbesondere die Dimere von $C_3$-$C_5$-Olefinen, sind Zwischenprodukte, die beispielsweise für die Herstellung von Aldehyden, Carbonsäuren und Alkoholen eingesetzt werden.

Werden n-Butene - das sind lineare Olefine mit vier Kohlenstoffatomen - einer Oligomerisierung unterworfen, entstehen dabei im Wesentlichen Olefine mit acht Kohlenstoffatomen (genauer gesagt: Dibuten) und dazu Olefine mit zwölf Kohlenstoffatomen (C12-Olefine, "Tributen"), sowie in einem geringeren Umfang Olefine mit mehr als zwölf Kohlenstoffatomen (C12+-Olefine). Die $C_8$-Olefine, die aus linearen Butenen entstehen, können durch Hydroformylierung und nachfolgender Hydrierung zu den entsprechenden Nonanolen umgesetzt werden, die wiederum vorwiegend für die Herstellung von Weichmachern Verwendung finden.

Die Oligomerisierung von Olefinen wird großtechnisch entweder in homogener Phase an einem gelösten oder heterogen an einem festen Katalysator oder mit einem zweiphasigen Katalysatorsystem durchgeführt.

Bei den heterogen katalysierten Verfahren ist die Oligomerisierung an sauren Kontakten lange bekannt. Technisch werden z. B. Zeolithe oder Phosphorsäure auf Trägern eingesetzt. Hierbei werden Isomerengemische von verzweigten Olefinen erhalten. Ein Beispiel für die saure Katalyse von Oligomerisierungen von Olefinen findet sich in der WO 92/13818.

Für die nicht-saure, heterogen katalysierte Oligomerisierung von Olefinen, mit hoher Dimerenselektivität werden in der Technik häufig Nickelverbindungen auf Trägermaterialien eingesetzt. Ein Katalysator dieser Art ist ein Nickel-Festbett-Katalysator, der im OCTOL-Prozess der Evonik Industries AG eingesetzt wird (Hydrocarbon Process., Int. Ed. (1988) 65 (2, Sect. 1, 31-33). Geträgerte Nickelkatalysatoren für diese Verwendung sind bekannt. So wird in der WO 95/14647 ein Nickelkatalysator mit einem Trägermaterial, das aus den Komponenten Titanoxid und/oder Zirkoniumoxid, Siliciumoxid und gegebenenfalls Aluminiumoxid und etwas Alkalioxid besteht, für die Olefinoligomerisierung beschrieben. An diesen Kontakten werden Gemische linearer Butene in einer Selektivität von unter 75 % zu $C_8$-Olefinen oligomerisiert.

Der Einsatz von Zeolithen als Trägermaterial hat sich durchaus als nachteilig erwiesen, da diese meist relativ große Poren und einen hohen Anteil an Protonen haben. Letztere führen zur säurekatalysierten Nebenreaktionen, insbesondere zur Bildung verzweigter Produkte.

Aufgabe der vorliegenden Erfindung war es, ein verbessertes Katalysatorsystem bereitzustellen, das die oben genannten Nachteile überwinden kann und insbesondere zu einer verbesserten umsatzabhängigen Selektivität für lineare Dimere beim Einsatz als Katalysator in der Oligomerisierung führen kann.

Überraschend wurde gefunden, dass das erfindungsgemäße Katalysatorsystem die Aufgabenstellung erfüllt.

Demgemäß ist ein erster Gegenstand der vorliegenden Erfindung ein Verfahren zur Oligomerisierung von Olefinen, wobei die Olefine gasförmig oder überkritisch vorliegen und wobei als Katalysator ein Ni-Ionen-getauschter Zeolith des Typs Linde Typ A eingesetzt wird.

Es hat sich überraschend gezeigt, dass durch Einsatz des spezifischen Ni-Ionen-getauschten kleinporigen Zeolithen des Typs Linde Typ A eine hohe Ausbeute an linearen Dimeren erhalten werden kann. Dies ist insofern überraschend, da gerade bei Zeolithen die Bildung eines hohen Anteils von verzweigten Produkten durch Säurekatalyse als typische Nebenreaktion auftritt.

Als Katalysator wird im Rahmen der vorliegenden Erfindung ein Ni-Ionen-getauschter Zeolith des Typs Linde Typ A (kurz LTA) eingesetzt. Zeolithe des Typs Linde Typ A werden vielfach auch kurz als Zeolith A bezeichnet. Sie basieren auf der chemischen Formel $|Na^+{}_{12} (H_2O)_{27}|_8 [Al_{12}Si_{12} O_{48}]_8$ und weisen eine kubische Einheitszelle auf mit einer verhältnismäßig kleinen Porenöffnung (8-MR). Eine genaue Beschreibung dieses Zeolith-Typen ist über die "International Zeolite Association" (www.iza-online.org) verfügbar. Die Synthese von Zeolithen des Typs Linde Typ A ist in H. Robson, K. P. Lillerud, Verified Synthesis of zeolitic materials, Second revised Edition, 2001, Elsevier, Seite 179 ff. beschrieben. Vorzugsweise wird ein LTA in seiner Kalium-, Natrium- oder Calcium- Form eingesetzt, insbesondere in seiner Natrium- oder Calcium- Form. Derartige Zeolithe sind kommerziell erhältlich, beispielsweise bei Sigma-Aldrich.

Zum Ionen-Austausch des Zeolithen gibt es an sich verschiedene Verfahren. Dabei werden die ursächlich im Zeolithen eingebundenen Kationen von Kalium, Natrium oder Calcium durch die gewünschten Kationen, beispielsweise Nickel, ausgetauscht. Dies ist an sich leicht möglich, da die Kationen im Zeolithen nur über eine ionische Wechselwirkung mit dem anionischen Gerüst verbunden sind. Ein Ionenaustausch durch Nickel kann beispielsweise mittels Flüssigionenaustausch aus einer entsprechenden Nickelsalz-haltigen Lösung erfolgen.

Im Rahmen der vorliegenden Erfindung erfolgt der Ionenaustausch insbesondere bevorzugt durch Flüssigionenaustausch.

In der einfachsten Ausführungsform der vorliegenden Erfindung wird der Natrium- oder Calciumbasierte LTA mit einer Nickelsalz-haltigen Lösung vermischt. Vorzugsweise handelt es sich bei der Nickelsalz-haltigen Lösung um eine wässrige Lösung von Nickelsalzen, insbesondere um das entsprechende Nitrat, Halogenid, Sulfat, Acetat, Citrat, Carbonat, wobei das Nitrat insbesondere bevorzugt ist.

Die Konzentration des Nickelsalzes in der Nickelsalz-haltigen Lösung liegt im Bereich von 0,001 Mol/l bis 2,5 Mol/l, insbesondere 0,005 Mol/l bis 0,5 Mol/l, wobei 0,01 Mol/l bis 0,1 Mol/l ganz besonders bevorzugt ist.

Der Flüssigionenaustausch mit Nickel erfolgt in der Regel über einen Zeitraum von 2 bis 48 Stunden, vorzugsweise 12 bis 36 Stunden, wobei 20 bis 28 Stunden ganz besonders bevorzugt werden. Dabei ist es vorteilhaft den Flüssigionenaustausch bei einer Temperatur von 60 bis 100 °C, vorzugsweise bei 80 °C vorzunehmen.

Im Anschluss kann der Katalysator nach dem Fachmann bekannten Methoden aufgearbeitet werden. Vorzugsweise wird der Katalysator nach dem Flüssigionenaustausch gewaschen, bevorzugt mit Wasser. Anschließend empfiehlt sich die Kalzinierung des Katalysators, insbesondere bei Temperaturen von 400 bis 600 °C, vorzugsweise bei Temperaturen um 500 °C. Die Kalzinierung kann dabei bevorzugt in Anwesenheit Luft erfolgen.

Der Anteil an Nickel im erfindungsgemäßen Katalysator liegt zwischen 1 und 10 Gew.-%, vorzugsweise bei 2 bis 8 Gew.-% und ganz besonders bevorzugt zwischen 4 und 7 Gew.-%. Das Nickel liegt dabei nicht in seiner metallischen Form vor, sondern als $Ni^{2+}$ in oxidischer Form, was nicht zwangsläufig stöchiometrisch vorliegen muss ($NiO_x$ mit $x \leq 1$).

In einer weiteren Ausführungsform der vorliegenden Erfindung kann zusätzlich ein zumindest partieller Austausch der im Zeolithen eingebundenen Kationen von Kalium, Natrium oder Calcium durch andere Alkali- und/oder Erdalkali-Ionen erfolgen. Bei den genannten Alkali- und/oder Erdalkali-Ionen handelt es sich insbesondere bevorzugt um Lithium und/oder Magnesium.

Im Rahmen der vorliegenden Erfindung hat sich überraschend herausgestellt, dass durch den zusätzlichen Austausch mit Lithium- und/oder Magnesium-Ionen der Umsatz erhöht und/oder die Isomerenstruktur der Dimere verbessert wird, wodurch das Gesamtverfahren wirtschaftlicher betrieben werden kann.

Vorzugsweise erfolgt der Austausch mit Alkali- und/oder Erdalkali-Ionen vor dem Flüssigionenaustausch mit Nickel. In der einfachsten Ausführungsform der vorliegenden Erfindung erfolgt auch der Austausch mit Alkali- und/oder Erdalkali-Ionen durch Flüssigionenaustausch. Dazu wird der eingesetzte LTA mit Lösungen der entsprechenden Alkali- und/oder Erdalkalisalze gemischt. Vorzugsweise handelt es sich um wässrige Lösungen der entsprechenden Alkali- und/oder Erdalkalisalze, wobei die entsprechenden Nitrate, Halogenide, Sulfate, Acetate, Citrate, Carbonate eingesetzt werden können, wobei Lösungen der entsprechenden Alkali- und/oder ErdalkaliHalogenide insbesondere bevorzugt sind. Ganz besonders bevorzugt werden Lithiumchlorid und Magnesiumchlorid eingesetzt.

Auch bei diesem Flüssigionenaustausch mit Alkali- und/oder Erdalkali-Ionen kann der Zeolith nach dem Fachmann bekannten Methoden aufgearbeitet werden. Vorzugsweise wird der Zeolith nach dem Flüssigionenaustausch gewaschen, ebenfalls bevorzugt mit Wasser. Anschließend empfiehlt sich auch hier die Kalzinierung des Katalysators, insbesondere bei Temperaturen von 400 bis 600 °C, vorzugsweise bei 500 °C. Die Kalzinierung kann dabei vorzugsweise in Anwesenheit von Luft erfolgen.

Der Anteil an mittels Flüssigionenaustausch eingebrachten Alkali- und/oder Erdalkali-Ionen im erfindungsgemäßen Katalysator liegt zwischen 0,02 und 8,8 Gew.-%, vorzugsweise zwischen 0,1 und 3 Gew.-% und ganz besonders bevorzugt zwischen 0,2 und 1,5 Gew.-%.

Als Eduktströme für das erfindungsgemäße Verfahren können Ströme, die C2- bis C10-, bevorzugt C2- bis C6-Olefine oder deren Gemische umfassen, eingesetzt werden. Geeignete Eduktströme können unter anderem α-Olefine, interne Olefine (beispielsweise 2-Olefine oder 3-Olefine) und Cycloalkene oder deren Gemische enthalten. In einer besonders bevorzugten Ausführungsform handelt es sich bei Eduktströmen um ein Gemisch aus Butenen, die n-Butan und/oder Isobutan, sowie kleine Anteile an anderen C4-Kohlenwasserstoffen enthalten können.

Bevorzugt enthält ein Eduktstrom wenig weitere ungesättigten Verbindungen und praktisch keine mehrfach ungesättigte Verbindungen wie Diene oder Acetylenderivate, vorzugsweise weniger als 100 Gew.-ppm in Bezug auf die Olefine im Eduktstrom. Weiterhin bevorzugt werden Olefingemische eingesetzt, die weniger als 5 Massen-%, insbesondere weniger als 2 Massen-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der $C_4$-Fraktion eines Steamcrackers gewonnen werden. Dabei wird im ersten Schritt Butadien entfernt. Dies geschieht entweder durch Extraktion oder Extraktionsdestillation des Butadiens oder dessen Selektivhydrierung. In beiden Fällen wird ein praktisch butadienfreier $C_4$-Schnitt erhalten, das Raffinat I. Im zweiten Schritt wird Isobuten aus dem $C_4$-Strom entfernt, z. B. durch Herstellung von Methyl-tert.-butylether (MTBE) durch Umsetzung mit Methanol. Andere Möglichkeiten sind die Umsetzung des Isobutens aus dem Raffinat I mit Wasser zu tert.-Butanol oder die sauer katalysierte Oligomerisierung des Isobutens zu Diisobuten. Der jetzt isobutenfreie $C_4$-Schnitt, das Raffinat II, enthält wie gewünscht die linearen Butene und gegebenenfalls Butane. Optional kann noch das 1-Buten destillativ abgetrennt werden. Beide Fraktionen, die mit But-1-en oder die mit But-2-en, können im erfindungsgemäßen Verfahren eingesetzt werden.

In einer weiteren bevorzugten Ausführungsform werden olefinhaltige Stoffströme wie Rohbutan dem Verfahren als Eduktstrom zugeführt. Weitere geeignete Eduktströme sind unter anderem das Raffinat I (butadienfreier C4-Schnitt des Steamcrackers) sowie das Raffinat II (butadien- und isobutenfreier C4-Schnitt des Steamcrackers).

Eine weitere Möglichkeit, einen geeigneten Eduktstrom herzustellen, besteht darin, Raffinat I, Raffinat II oder ein ähnlich zusammengesetztes Kohlenwasserstoffgemisch in einer Reaktivkolonne zu hydroisomerisieren. Dabei kann u. a. ein Gemisch gewonnen werden, das aus 2-Butenen, geringen Anteilen 1-Buten und gegebenenfalls n-Butan sowie Isobutan und Isobuten besteht.

Weiterer Gegenstand der vorliegenden Erfindung ist ein Katalysatorsystem umfassend einen Ni-Ionen-getauschten Zeolithen des Typs Linde Typ A mit einem Gehalt an Ni von 1 bis 10 Gew.-% (bezogen auf das Gesamtgewicht Katalysator).

In einer besonders bevorzugten Ausführungsform handelt es sich bei dem erfindungsgemäßen Katalysator um einen Ni-Ionen-getauschten Zeolithen des Typs Linde Typ A mit einem Gehalt an Ni von 1 bis 10 Gew.-%, vorzugsweise 4 bis 7 Gew.-% (bezogen auf das Gesamtgewicht des Katalysators) und einem Gehalt an Alkali- oder Erdalkali-Ionen zwischen 0,02 und 8,8 Gew.-%, vorzugsweise zwischen 0,1 und 3 Gew.-% und ganz besonders bevorzugt zwischen 0,2 und 1,5 Gew.-%. Besonders Bevozugt sind Gehalte im Bereich von 0,2 Gew.-% für Lithium und/oder von 1,5 Gew.-% für Magnesium (bezogen auf das Gesamtgewicht des Katalysators).

Gegenstand der Erfindung ist weiterhin ein Verfahren zur Oligomerisierung von n-Buten, umfassend die folgenden Schritte

    A) Bereitstellen eines erfindungsgemäßen Katalysatorsystems,

    B) Oligomerisierung von n-Buten durch Kontaktieren des Katalysatorsystems mit einem n-Butenhaltigen Gemisch.

Entsprechende Quellen für n-Buten sind bereits vorab genannt.

In einer Ausführungsform beträgt der Anteil an Dimeren (auch "prozentuale Selektivität bezogen auf die Dimerisierung" genannt) bezogen auf das umgesetzte Edukt mindestens 60 %, weiter bevorzugt mindestens 85 %, besonders bevorzugt mindestens 90 %, insbesondere mindestens 95 %. In einer besonders bevorzugten Ausführungsform beträgt der Anteil an Dimeren mindestens 96 %, weiter bevorzugt mindestens 98 %, besonders bevorzugt mindestens 99 %.

Die nach dem erfindungsgemäßen Verfahren hergestellten Oligomeren werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein $C_9$-Alkoholgemisch. Das $C_9$-Säuregemisch kann zur Herstellung von Schmiermitteln oder Sikkative verwendet werden. Das $C_9$-Alkoholgemisch ist Vorstufe für die Herstellung von Weichmachern, insbesondere von Di-Nonyl-phthalaten. Gerade für die optimalen Eigenschaften der Weichmacher spielt die möglichst hohe Linearität der C9-Alkohole und damit auch die Linearität der dafür notwendigen C8-Olefine eine entscheidende Rolle. Die Linearität wird üblicherweise über den ISO-Index bestimmt.

Die Linearität der Dimerenfraktion wird durch den ISO-Index beschrieben und stellt einen Wert für die mittlere Anzahl an Methylverzweigungen im Dimer dar. So tragen z.B. n-Oktene mit 0, Methylheptene mit 1 und Dimethylhexene mit 2 zum ISO-Index einer C8-Fraktion bei. Je niedriger der ISO-Index ist, um so linearer sind die Moleküle in der jeweiligen Fraktion aufgebaut. Der ISO-Index errechnet sich nach folgender allgemeiner Formel:

$$\frac{\text{einfach verzweigte Dimere (Gew.-\%)} + 2 \times \text{zweifach verzeigte Dimere (Gew.-\%)}}{100}$$

Demnach besitzt ein Dimerengemisch mit einem ISO-Index von 1,0 im Mittel genau eine Methylverzweigung pro Dimerenmolekül. ISO-Indices um 1,0 stellen den Standard in Ni-haltigen Katalysatorsystemen dar. Weiterhin ist bekannt, dass diese ISO-Indices umsatzabhängig sind, d.h. bei sinkendem Umsatz per pass (also der Umsatz im geraden Durchgang durch das Katalysatorbett) wird die Dimerfraktion linearer. Da aufgrund wirtschaftlicher Erwägungen der Umsatz aber nicht beliebig klein gewählt werden kann, muss immer ein Kompromiss aus Umsatzleistung (Raum-Zeit-Ausbeute, Produktmasse pro Katalysatormasse und Zeit) und Linearität der Produkte gewählt werden.

Es wurden nun aber erfindungsgemäße Katalysatorsysteme gefunden, bei deren Einsatz bei der Oligomerisierung von Olefinen die daraus erhaltene Produktmischung (Dimerenfraktion) einen ISO-Index von deutlich kleiner 1 aufweisen ohne dass der Umsatz per pass deutlich kleiner ausfällt als üblich. Selbst bei Umsätzen per pass von >10%, bevorzugt >15%, besonders bevorzugt >20% werden mit den erfindungsgemäßen Katalysatorsystemen im Oligomerisierungsverfahren ISO-Indices von kleiner 0,8 erzielt, bevorzugt kleiner 0,7, ganz besonders bevorzugt kleiner 0,65 erreicht.

Die Oligomerisierung erfolgt in der Regel bei einer Temperatur im Bereich von 150 bis 180 °C, bevorzugt im Bereich von 155 bis 170°C, und bei einem Druck von 40 bis 60 bar, bevorzugt von 45 bis 55 bar. Die gewichtbasierten Raumgeschwindigkeiten (Reaktandmasse pro Katalysatormasse pro Zeit; weight hourly space velocity (WHSV)) befinden sich im Bereich zwischen 1,5 g Reaktand pro g Katalysator und pro h ($h^{-1}$) und 1900 $h^{-1}$, vorzugsweise zwischen 4 $h^{-1}$ und 350 $h^{-1}$, besonders bevorzugt zwischen 6 $h^{-1}$ und 125 $h^{-1}$.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

### Beispiele

### Katalysatorsynthese:

Zeolith des Typs Linde Typ A (LTA) in der Natrium- oder Calciumform (Fa. Sigma-Aldrich) wurde 4 h bei 500 °C vorkalziniert (Aufheizgeschwindigkeit: 5 °C/min, im Luftstrom mit 100 ml/min Luft) und entweder direkt mit Nickelionen ausgetauscht verwendet ("Ni auf Na-LTA" und "Ni auf Ca-LTA") oder wurde vor dem Nickelaustausch mit anderen Co-Kationen ausgetauscht. Für diesen Austausch wurde der Zeolith viermal mit einer 0,5 M wässrigen Lösung von LiCl oder $MgCl_2$; (20 g/g Zeolith) gemischt und jeweils vier Stunden bei 80 °C gerührt. Nach jedem Austauschschritt wurde die Flüssigkeit durch Zentrifugation entfernt und eine frische Lösung zugegeben. Nach dem letzten Austauschschritt wurde der Zeolith mit entionisiertem Wasser (2 l) gewaschen, über Nacht getrocknet und kalziniert (8 Stunden bei 500 °C, Aufheizgeschwindigkeit: 5 °C/min, im Luftstrom mit 100 ml/min Luft).

Für den Nickelaustausch wurde der LTA-Precursor mit einer wässrigen Lösung von Nickelnitrat mit Konzentrationen von 0,01 bis 0,1 M (20 g/g Katalysator) gemischt und für 24 h bei 80 °C gerührt. Anschließend wurde der Katalysatorvorläufer mit Wasser (2 l) gewaschen, getrocknet und kalziniert (8 h bei 500 °C, Aufheizgeschwindigkeit: 5 °C/min, im Luftstrom mit 100 ml/min Luft).

### Reaktionsprozess:

Vor dem Beladen in den Reaktor wurde der Katalysator für mindestens 1 h bei 100 °C getrocknet. Danach wurden zwischen 10 und 200 mg des Katalysators mit Siliciumcarbid (SiC) verdünnt, um ein Gesamtgewicht von 800 mg zu erreichen. Diese Mischung wurde in der Mitte eines 30 cm langen Rohrreaktors mit einem Innendurchmesser von 0,152 Zoll installiert. SiC wurde verwendet, um das Katalysatorbett zu fixieren. Der Katalysator wurde in strömender Luft (100 ml / min) für 2 Stunden bei 450 °C (Aufheizgeschwindigkeit: 10 °C / min) aktiviert. Nach dem Spülen mit $N_2$ wurde der Ansatz mit $N_2$ beaufschlagt und anschließend mit der Eduktmischung (85 % 1-Buten, 15 % i-Butan) über eine Spritzenpumpe (ISCO SYRINGE PUMP 500 D; inklusive Kühlaggregat zur Aufrechterhaltung von 14 °C) versetzt. Die gewünschte Durchflussmenge wurde eingestellt (0,03-0,2 ml / min) und die Erwärmung wurde gestartet. Die Produkte, die im geraden Durchgang durch das Katalysatorbett gebildet wurden, wurden mittels Online-GC analysiert. Vor der GC-Injektion wurden 100 ml / min $H_2$ zugegeben, um den Produktstrom über einen Pt / $Al_2O_3$-Katalysator bei Umgebungsdruck zu hydrieren.

Ergebnisse:

**6 Gew.-% Ni auf Na-LTA**

| Umsatz / % | WHSV / g*g⁻¹*h⁻¹ | Raum-Zeit-Ausbeute / g*g⁻¹*h⁻¹ | $C_8$ Selektivität / % | | | | Oligomer Selektivität / % | | |
|---|---|---|---|---|---|---|---|---|---|
| | | | Dimethylhexen | Methylhepten | Okten | ISO-Index | C8 | C12 | C16 |
| 12,8 | 4,3 | 0,6 | 3.0 | 46.1 | 50.8 | 0.52 | 89.5 | 10.5 | 0.0 |
| 10,5 | 6,4 | 0,7 | 0.0 | 45.5 | 54.5 | 0.46 | 100.0 | 0.0 | 0.0 |
| 6,4 | 12,8 | 0,8 | 0.0 | 40.9 | 59.1 | 0.41 | 100.0 | 0.0 | 0.0 |
| 1,9 | 25,5 | 0,5 | 0.0 | 37.0 | 63.0 | 0.37 | 100.0 | 0.0 | 0.0 |

| 6 Gew-% Ni auf Li-Na-LTA | | | | | | | Oligomer Selektivität / % | | |
|---|---|---|---|---|---|---|---|---|---|
| Umsatz / % | WHSV/ $h^{-1}$ | Raum-Zeit-Ausbeute / $g*g^{-1}*h^{-1}$ | Dimethylhexen | Methylhepten | Okten | ISO-Index | C8 | C12 | C16 |
| 10,5 | 4,3 | 0,5 | 1.8 | 41.7 | 56.5 | 0.45 | 86.0 | 14.0 | 0.0 |
| 6,4 | 6,4 | 0,4 | 1.6 | 39.2 | 59.2 | 0.42 | 87.6 | 12.4 | 0.0 |
| 4,3 | 12,8 | 0,6 | 0.0 | 39.0 | 61.0 | 0.39 | 89.2 | 10.8 | 0.0 |

| 6 Gew-% Ni auf Mg-Na-LTA | | | | | | | Oligomer Selektivität / % | | |
|---|---|---|---|---|---|---|---|---|---|
| Umsatz / % | WHSV/ $h^{-1}$ | Raum-Zeit-Ausbeute / $g*g^{-1}*h^{-1}$ | Dimethylhexen | Methylhepten | Okten | ISO-Index | C8 | C12 | C16 |
| 20,0 | 12,8 | 2,6 | 4.7 | 50.9 | 44.4 | 0.60 | 88.3 | 11.7 | 0.0 |
| 16,3 | 25,8 | 4,2 | 4.1 | 47.5 | 48.5 | 0.56 | 88.8 | 11.2 | 0.0 |

| 6 Gew-% Ni auf Ca-LTA | | | | | | | Oligomer Selektivität / % | | |
|---|---|---|---|---|---|---|---|---|---|
| Umsatz / % | WHSV/ $h^{-1}$ | Raum-Zeit-Ausbeute / $g*g^{-1}*h^{-1}$ | Dimethylhexen | Methylhepten | Okten | ISO-Index | C8 | C12 | C16 |
| 34,5 | 6,4 | 2,2 | 4.9 | 54.8 | 40.2 | 0.65 | 84.0 | 16.0 | 0.0 |
| 28,6 | 12,8 | 3,7 | 4.4 | 52.0 | 43.6 | 0.61 | 85.1 | 14.9 | 0.0 |
| 24,0 | 25,5 | 6,1 | 3.3 | 47.0 | 49.7 | 0.54 | 82.9 | 15.1 | 2.0 |
| 19,0 | 38,1 | 7,2 | 2.4 | 44.2 | 53.4 | 0.49 | 87.8 | 12.2 | 0.0 |
| 12,8 | 101,5 | 13,0 | 2.4 | 37.3 | 47.1 | 0.48 | 86.6 | 13.0 | 0.4 |

| 6 Gew-% Ni auf Li-Ca-LTA | | | | | | | Oligomer Selektivität / % | | |
|---|---|---|---|---|---|---|---|---|---|
| Umsatz / % | WHSV/ $h^{-1}$ | Raum-Zeit-Ausbeute / $g*g^{-1}*h^{-1}$ | Dimethylhexen | Methylhepten | Okten | ISO-Index | C8 | C12 | C16 |
| 37,3 | 6,4 | 2,42 | 5.5 | 53.7 | 40.8 | 0.65 | 83.9 | 16.1 | 0.0 |
| 35,2 | 12,7 | 4,5 | 4.4 | 49.9 | 45.8 | 0.59 | 83.6 | 16.4 | 0.0 |
| 32,9 | 25,6 | 8,4 | 5.1 | 50.3 | 44.6 | 0.61 | 82.2 | 15.7 | 2.2 |
| 20,4 | 102,4 | 20,9 | 3.6 | 45.4 | 51.0 | 0.53 | 88.0 | 11.1 | 0.9 |

| 6 Gew-% Ni auf Mg-Ca-LTA | | | | | | | Oligomer Selektivität / % | | |
|---|---|---|---|---|---|---|---|---|---|
| Umsatz / % | WHSV/ $h^{-1}$ | Raum-Zeit-Ausbeute / $g*g^{-1}*h^{-1}$ | Dimethylhexen | Methylhepten | Okten | ISO-Index | C8 | C12 | C16 |
| 24,8 | 25,6 | 6,3 | 8.3 | 54.8 | 36.9 | 0.71 | 89.6 | 10.4 | 0.0 |
| 22,8 | 50,7 | 11,6 | 7.7 | 53.1 | 39.2 | 0.69 | 89.4 | 10.6 | 0.0 |

Zusammenfassend zeigen die vorliegenden Ergebnisse, dass Ni-Ionen-getauschte Zeolithen des Typs Linde Typ A in der Buten-Dimerisierung hoch aktiv sind und eine hohe Selektivität gegenüber linearen und monoverzweigten Dimeren aufweisen. Sowohl die Aktivität als auch die Selektivität können durch die Menge und die Art der Co-Kationen abgestimmt werden. Dabei ist immer auf die spezifische Produktleistung (auch: Raum-Zeit-Ausbeute) abzustellen, das heißt eine Gesamtbetrachtung wie viel Produkt pro Raum und Zeit in einem Reaktor gebildet werden. Hohe Selektivitäten bei kleinen WHSV und/oder kleinen Umsätzen sind aus wirtschaftlicher Sicht deswegen keineswegs besser, da pro Zeiteinheit weniger Produkt gebildet wird, als bei einer vergleichbaren Reaktion mit geringere Selektivität bei höhere WHSV und/oder bei höherem Umsatz. Dabei zeigt sich dass insbesondere die mit anderen Alkali- oder Erdalkali-Ionen ausgestauschten erfindungsgemäßen Zeolithe des Typs Linde A eine besonderes gute Kombination von hohen Selektivitäten (Iso-Indices) mit gleichzeitig hohen Raum-Zeit-Ausbeuten.

Diese Anwendung eines spezifischen Zeoliths für eine hochselektive Olefinoligomerisierung ist dem Fachmann bislang nicht bekannt.

## Patentansprüche

1. Katalysatorsystem umfassend einen Ni-Ionen-getauschten Zeolithen des Typs Linde Typ A mit einem Gehalt an Ni von 1 bis 10 Gew.%-(bezogen auf die Gesamtmasse des Katalysators), **dadurch gekennzeichnet, dass** zusätzlich ein zumindest partieller Austausch der im Zeolithen eingebundenen Kationen von Kalium, Natrium oder Calcium durch andere Alkali- oder Erdalkali-Ionen erfolgt ist, wobei der Anteil an eingebrachten Alkali- und/oder Erdalkali-Ionen zwischen 0,02 und 8,8 Gew.-% liegt.

2. Katalysatorsystem gemäß Anspruch 1, **dadurch gekennzeichnet, dass** der Austausch mit Ni-Ionen durch Flüssigionenaustausch erfolgt ist.

3. Katalysatorsystem gemäß Anspruch 1 oder 2, **dadurch gekennzeichnet, dass** die Alkali- oder Erdalkali-Ionen ausgewählt sind aus Lithium und/oder Magnesium.

4. Katalysatorsystem gemäß einem oder mehreren der Ansprüche 1 bis 3, **dadurch gekennzeichnet, dass** der Austausch mit Alkali- und/oder Erdalkali-Ionen durch Flüssigionenaustausch erfolgt ist.

5. Verfahren zur Oligomerisierung von Olefinen, wobei die Olefine gasförmig oder überkritisch vorliegen, wobei als Katalysator ein Ni-Ionen-getauschter Zeolith des Typs Linde Typ A mit einem Gehalt an Ni von 1 bis 10 Gew.-(bezogen auf die Gesamtmasse des Katalysators) eingesetzt wird und wobei bei einem Umsatz per pass von >10% ein ISO-Index von kleiner 0,8 erzielt wird.

6. Verfahren gemäß Anspruch 5, **dadurch gekennzeichnet, dass** als Olefine C2- bis C6-Olefine oder deren Gemische eingesetzt werden.

7. Verfahren gemäß Anspruch 5 oder 6, **dadurch gekennzeichnet, dass** es sich bei dem Olefin um n-Buten handelt.

8. Verfahren zur Oligomerisierung von n-Buten, umfassend die folgenden Schritte

   A) Bereitstellen eines Katalysatorsystems gemäß Anspruch 1
   B) Oligomerisierung von n-Buten durch Kontaktieren des Katalysatorsystems mit einem n-Butenhaltigen Gemisch.

9. Verfahren gemäß Anspruch 8, **dadurch gekennzeichnet, dass** die Oligomerisierung bei einer Temperatur im Bereich von 150 bis 180 °C und bei einem Druck von 40 bis 60 bar erfolgt.

Europäisches
Patentamt

European
Patent Office

Office européen
des brevets

**EUROPÄISCHER RECHERCHENBERICHT**

Nummer der Anmeldung

EP 18 15 8148

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | By A Tungler ET AL: "MAGNETIC STUDY OF. ZEOLITE-SUPPORTED NICKEL CATALYSTS", , 1. Januar 1978 (1978-01-01), Seiten 319-325, XP055395259, Gefunden im Internet: URL:http://acta.bibl.u-szeged.hu/24086/1/phys_chem_024_fasc_001_002_319-325.pdf [gefunden am 2017-08-01] * page 320, table 1, first entry * ----- | 1-9 | INV. C01B39/14 C07C2/12 B01J29/76 |
| Y | K. KLIER ET.AL.: "Spectra of zynthetic zeolites containing transition metal ions-II. Ni2+ ions in type A Linde molecular sieves", J. PHYS. CHEM. SOLIDS, Bd. 29, 1968, Seiten 951-957, XP002772731, * page 952, left column, lines 22-40 * ----- | 1-9 | |
| Y | MARK A. DEIMUND ET.AL.: "Nickel-exchanged zincosilicate catalysts for t he oligomerization of propylene", ACS CATAL., Bd. 4, 14. Oktober 2014 (2014-10-14), Seiten 4189-4195, XP002772732, * page 4189, left column, lines 1-4; scheme 1 * ----- | 1-9 | **RECHERCHIERTE SACHGEBIETE (IPC)** C01B C07C B01J |
| Y | B. NKOSI ET.AL.: "The oligomerization of butenes with partially alkali exchanged NiNaY zeolite catalysts", APPLIED CATALYSIS A: GENERAL, Bd. 158, 1997, Seiten 225-241, XP002772733, * page 232, paragraph 3.2 - page 235, line 15; Abbildung 3; Tabellen 1-4 * ----- | 1-9 | |

Der vorliegende Recherchenbericht wurde für alle Patentansprüche erstellt

| Recherchenort | Abschlußdatum der Recherche | Prüfer |
|---|---|---|
| München | 20. Juni 2018 | Kleidernigg, Oliver |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
   Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9213818 A

- WO 9514647 A

**In der Beschreibung aufgeführte Nicht-Patentliteratur**

- *Hydrocarbon Process., Int. Ed.,* 1988, vol. 65, 31-33

- **H. ROBSON ; K. P. LILLERUD.** Verified Synthesis of zeolitic materials. Elsevier, 2001, 179